# EUROPEAN PATENT APPLICATION

(11) **EP 2 687 836 A1**
(43) Date of publication of application: **22.01.2014**
(21) Application number: 12761266.1
(22) Date of filing: 05.03.2012
(51) Int. Cl.: G01N 21/78, G01N 33/497

(54) **NITROGEN OXIDE CONCENTRATION MEASUREMENT DEVICE**

(30) Priority: 18.03.2011 JP 2011060414
(71) Applicant: Panasonic Healthcare Co., Ltd., Ehime 791-0395 (JP)
(72) Inventor: MIKI, Hideshi, Chuo-ku, Osaka 540-6207 (JP); TANAKA, Motohiro, Chuo-ku, Osaka 540-6207 (JP); MATSUBARA, Fumiya, Vhuo-ku, Osaka 540-6207 (JP)
(74) Representative: Eisenführ Speiser
(86) International application number: PCT/JP2012/001512
(87) International publication number: WO 2012/127794

(57) **Abstract**

A nitrogen oxide concentration measurement device (10) comprises a zero scrubber (13) disposed within a gas flow path (16), and a controller (28). The zero scrubber (13) performs subjects exhaled air flowing through the gas flow path (16) from a mouthpiece (11) toward a chamber (15) to a NO removal treatment in a first period, out of a first period and a second period. The controller (28) acquires the concentration of NO contained in the exhaled air on the basis of the differential voltage value (Vd) outputted in the second period, which uses as a reference the differential voltage value (Vd) outputted in the first period.

## Description

### TECHNICAL FIELD

The present invention relates to a nitrogen oxide concentration measurement device for measuring the concentration of nitrogen oxides, such as nitric oxide.

### BACKGROUND ART

Ever since nitric oxide (hereinafter referred to as "NO") was discovered as the main ingredient of a muscle relaxing factor, and its physiological action was revealed, NO concentration has been measured and utilized a neural information transmitter or an infection marker.

In particular, it has been noted that the result of measuring NO concentrations in exhaled air can be used as an index of the extent of respiratory tract infection due to allergies and asthma, which has been on the rise in recent years, making possible a non-invasive diagnosis of disease that is less of a burden to the patient. The NO gas concentration in exhaled air is from 2 to 20 ppb in healthy people, but it is known that it can roughly triple in patients with airway inflammation due to asthma or allergies. Accordingly, if exhaled NO gas is measured, the result can be used as a guideline for treating asthma, such as in determining the extent of airway inflammation in the patient, or deciding on dosages of asthma drugs.

Patent Literature 1 discusses a method in which the nitrogen oxide concentration is measured by using a detecting element that contains porphyrin, which reacts with NO. More specifically, with the method discussed in Patent Literature 1, the NO concentration is measured as follows. First, a voltage value output component outputs a voltage value indicating the change in the amount of reflected light caused by the detecting element in a first period, in which the detecting element is exposed to a gas not containing any NO, and a second period, which follows the first period and in which the detecting element is exposed to exhaled air. Next, a controller acquires a first voltage value outputted in the first period and a second voltage value outputted in the second period, and subtracts the first voltage value from the second voltage value to perform zero point correction of the second voltage value. The controller then calculates the concentration of NO contained in the exhaled air on the basis of the second voltage value that has undergone zero point correction.

### CITATION LIST

### PATENT LITERATURE

Patent Literature 1: WO2010/061536

### SUMMARY

### TECHNICAL PROBLEM

Patent Literature 1 makes no particular mention of the method for producing a gas that does not contain NO and that is supplied in the first period, but an example of a possible method is a simple production method in which air taken in from outside the device is subjected to a NO removal treatment. With such a method, however, there is the risk that exhaled air components during measurement will cause fluctuations in the gas components after the NO removal treatment, so a problem is that the concentration of NO contained in exhaled air cannot be measured accurately.

The present invention was conceived in light of the above problem, and it is an object thereof to provide a nitrogen oxide concentration measurement device with which the concentration of NO contained in exhaled air can be measured accurately.

### SOLUTION TO PROBLEM

The nitrogen oxide concentration measurement device pertaining to a first aspect comprises a detecting element, a mouthpiece, a chamber, a gas flow path, a NO removal unit, a light source, a voltage value output component, and a controller. The detecting element contains a metal complex of porphyrin or a derivative thereof. The mouthpiece is used to take in exhaled air. The chamber houses the detecting element. The gas flow path communicates with the mouthpiece and the chamber. The NO removal unit is disposed within the gas flow path, and subjects exhaled air flowing through the gas flow path from the mouthpiece toward the chamber to a NO removal treatment in a first period, out of a first period and a second period that follows the first period. The light source shines light on the detecting element during the first and second periods. The voltage value output component outputs, during the first and second periods, a voltage value indicating the amount of light having a specific wavelength out of all the light emitted from the detecting element. The controller acquires the concentration of NO contained in the exhaled air on the basis of the voltage value outputted from the voltage value output component.

### ADVANTAGEOUS EFFECTS

The present invention provides a nitrogen oxide concentration measurement device with which the concentration of NO contained in exhaled air can be measured accurately.

### BRIEF DESCRIPTION OF DRAWINGS

FIG. 1 is a block diagram showing the simplified configuration of a nitrogen oxide concentration measurement device;
FIG. 2 is a block diagram showing the simplified internal configuration of a nitrogen oxide concentration measurement device;
FIG. 3 shows the structure of a metal complex of a porphyrin derivative (CoT(dtBu)PP) supported on a detecting element used in a nitrogen oxide concentration measurement device;
FIG. 4 is a graph of the absorption spectrum of CoT(dtBu)PP;
FIG. 5 is a function block diagram of the configuration of a controller;
FIG. 6 is a graph of an example of a differential voltage value curve showing the transition in differential voltage values;
FIG. 7 is a graph of an example of a baseline; and
FIG. 8 is a graph of an example of a baseline and an example of a differential voltage value curve showing the transition in differential voltage values.

### DESCRIPTION OF EMBODIMENTS

Next, an embodiment of the present invention will be described through reference to the drawings. In the following discussion of the drawings, those portions that are the same or similar will be given the same or similar numbers. The drawings, however, are merely schematic in nature, and the dimensional proportions and so forth may differ from those in actuality. Therefore, the following description should be referred to for specific dimensions and the like. Also, dimensional relations and proportions may of course vary from one drawing to another.

### Simplified Configuration of Nitrogen Oxide Concentration Measurement Device 10

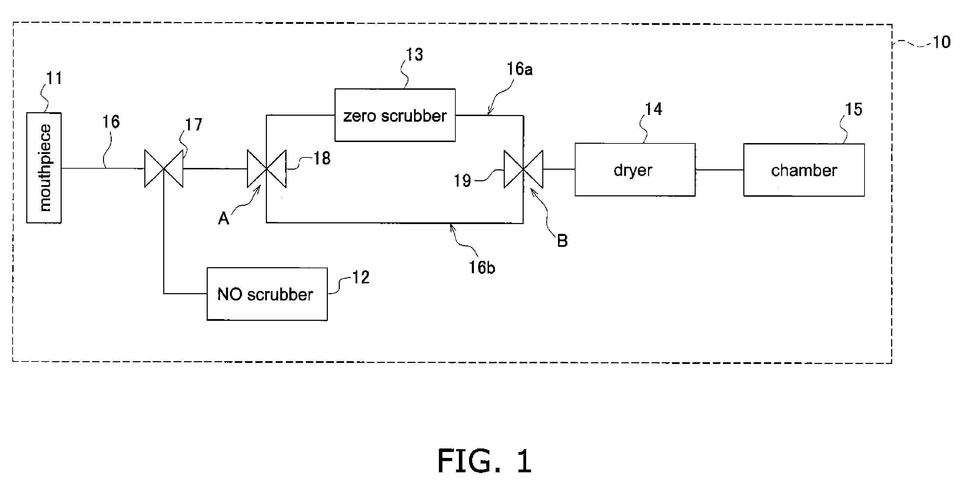

The simplified configuration of the nitrogen oxide concentration measurement device 10 pertaining to an embodiment will be described through reference to the drawings. FIG. 1 is a block diagram showing the simplified configuration of the nitrogen oxide concentration measurement device 10 pertaining to this embodiment.

The nitrogen oxide concentration measurement device 10 is used to measure the concentration of nitric oxide (hereinafter referred to as "NO") contained in the exhaled air from an asthma patient or the like (hereinafter referred to as "user"). The nitrogen oxide concentration measurement device 10 comprises a mouthpiece 11, an NO scrubber 12, a zero scrubber 13, a dryer 14, a chamber 15, a gas flow path 16, an upstream valve 17, a mid-stream valve 18, and a downstream valve 19.

The mouthpiece 11 is used to take in exhaled air from the user. The user places the mouthpiece 11 against his mouth and exhales into the nitrogen oxide concentration measurement device 10.

The NO scrubber 12 has air holes (not shown) for taking in outside air. The NO scrubber 12 contains a material having the property of removing NO (such as potassium permanganate or activated charcoal). The NO scrubber 12 subjects the air brought in through the air holes to a NO removal treatment.

The zero scrubber 13 (an example of a "NO removal unit") is disposed downstream of the mouthpiece 11 within the gas flow path 16. The zero scrubber 13 contains a material having the property of removing NO (such as potassium permanganate or activated charcoal). The zero scrubber 13 subjects the exhaled air brought in from the mouthpiece 11 to the NO removal treatment. More specifically, the zero scrubber 13 subjects the exhaled air to the NO removal treatment in a first period (discussed below), and does not subject the exhaled air to the NO removal treatment in a second period (discussed below). The zero scrubber 13 also subjects the exhaled air to the NO removal treatment in a third period (discussed below).

The dryer 14 is disposed upstream of the chamber 15 within the gas flow path 16. The dryer 14 contains a material having a desiccating property (such as silica gel). The dryer 14 subjects the exhaled air brought into the chamber 15 (including exhaled air from which NO has already been removed) to a desiccation treatment.

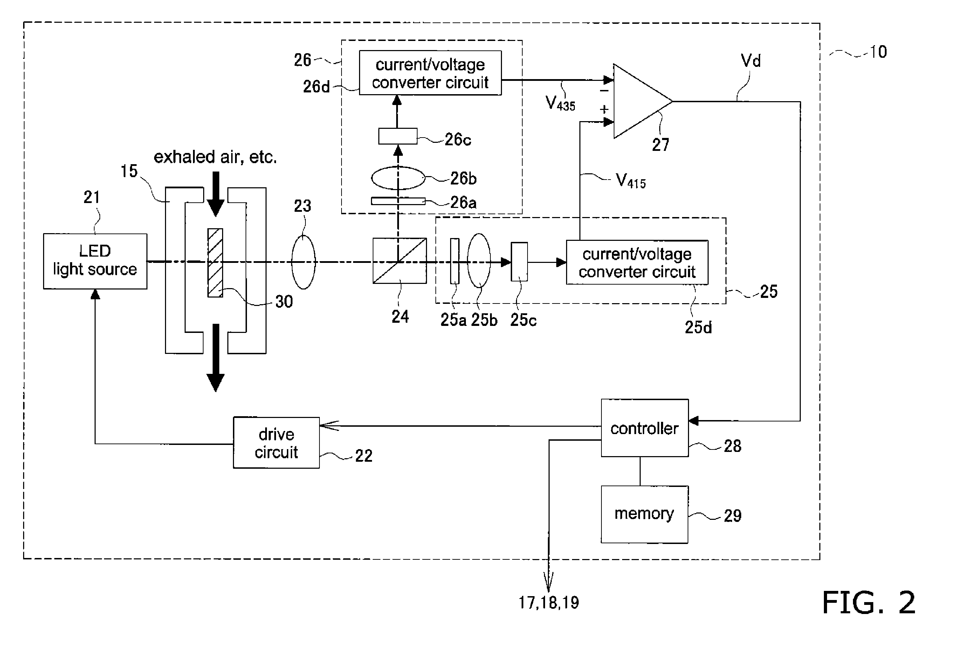

The chamber 15 is a case in which a detecting element 30 is installed. The chamber 15 has a structure that transmits the light emitted by an LED light source 21 (discussed below; see FIG. 2).

The gas flow path 16 is linked to the mouthpiece 11 and the NO scrubber 12. The gas flow path 16 is also linked to the mouthpiece 11 and the chamber 15. The gas flow path 16 is constituted by a cylindrical flue, for example. The gas flow path 16 includes a first flow path 16a and a second flow path 16b. The first flow path 16a is formed between the mouthpiece 11 and the chamber 15. The first flow path 16a houses the zero scrubber 13. The second flow path 16b is formed between the mouthpiece 11 and the chamber 15, and is linked to both ends of the first flow path 16a.

The upstream valve 17 is a two-position valve. The upstream valve 17 is disposed downstream of the mouthpiece 11 within the gas flow path 16. The upstream valve 17 switches what the mouthpiece 11 is linked to, under position switching control performed by a controller 28 (discussed below; see FIG. 2). More specifically, the upstream valve 17 blocks off the flow path going toward the chamber 15 while the user is inhaling air from the outside, and blocks off the flow path going toward the NO scrubber 12 while the user is exhaling.

The mid-stream valve 18 (an example of a first flow path switching component) is a two-position valve. The mid-stream valve 18 is disposed at a branch point A between the first flow path 16a and the second flow path 16b within the gas flow path 16. The mid-stream valve 18 blocks off the first flow path 16a or the second flow path 16b under position switching control by the controller 28. More specifically, the mid-stream valve 18 blocks off the second flow path 16b, the first flow path 16a, and the second flow path 16b, in that order, when the user exhales.

The downstream valve 19 (an example of a second flow path switching component) is a two-position valve. The downstream valve 19 is disposed at a merge point B between the first flow path 16a and the second flow path 16b within the gas flow path 16. The downstream valve 19 blocks off the second flow path 16b, the first flow path 16a, and the second flow path 16b, in that order, just as does the mid-stream valve 18, under position switching control by the controller 28.

### Internal Configuration of Nitrogen Oxide Concentration Measurement Device 10

The internal configuration by which the nitrogen oxide concentration measurement device 10 pertaining to this embodiment measures NO concentration will be described through reference to the drawings. FIG. 2 is a block diagram showing the simplified internal configuration of the nitrogen oxide concentration measurement device 10 pertaining to this embodiment.

The nitrogen oxide concentration measurement device 10 comprises the LED light source 21, a drive circuit 22, a lens 23, a dichroic mirror 24, a 415 nm light quantity detector 25, a 435 nm light quantity detector 26, a differential computation processor 27, the controller 28, and a memory 29.

The LED light source 21 (an example of a "light source") shines light under specific conditions at the detecting element 30 installed in the chamber 15. The configuration of the detecting element 30 will be discussed below.

The drive circuit 22 adjusts the amount of light directed at the detecting element 30 by varying the amount of current supplied to the LED light source. In this embodiment, the drive circuit 22 is configured so that at least two types of current are applied during initialization and during measurement.

The lens 23 and the dichroic mirror 24 are disposed in that order between the chamber 15 and the 415 nm light quantity detector 25 and 435 nm light quantity detector 26. The dichroic mirror 24 reflects light with a wavelength near 435 nm (hereinafter referred to as "435 nm light") out of all the light transmitted from the detecting element 30 and received via the lens 23, and transmits light with any other wavelength.

The 415 nm light quantity detector 25 is disposed along the path of light transmitted by the dichroic mirror 24. As shown in FIG. 2, the 415 nm light quantity detector 25 comprises a band pass filter 25a, a lens 25b, a photodiode 25c, and a current/voltage converter circuit 25d.

The band pass filter 25a is an optical filter that transmits only light with a wavelength near 415 nm (hereinafter referred to as "415 nm light"). Specifically, the band pass filter 25a guides only the 415 nm light transmitted by the dichroic mirror 24 to the lens 25b. The lens 25b guides the 415 nm light transmitted by the band pass filter 25a to the photodiode 25c. The photodiode 25c outputs current corresponding to the amount of 415 nm light (hereinafter referred to as "415 nm light quantity") transmitted by the lens 25b. The current/voltage converter circuit 25d converts the current value outputted from the photodiode 25c into a voltage value, and outputs this to the differential computation processor 27. With the above configuration, the 415 nm light quantity detector 25 outputs a voltage value V₄₁₅ pertaining to the 415 nm light quantity.

The 435 nm light quantity detector 26 is disposed along the path of the 435 nm light reflected by the dichroic mirror 24. As shown in FIG. 2, the 415 nm light quantity detector 25 comprises the band pass filter 26a, a lens 26b, a photodiode 26c, and a current/voltage converter circuit 26d.

The band pass filter 26a transmits only 435 nm light. The lens 26b guides the light transmitted by the band pass filter 26a to the photodiode 26c. The photodiode 26c outputs current corresponding to the amount of 435 nm light transmitted by the lens 26b (hereinafter referred to as "435 nm light quantity"). The current/voltage converter circuit 26d converts the current value outputted from the photodiode 26c into a voltage value, and outputs this to the differential computation processor 27. With the above configuration, the 435 nm light quantity detector 26 outputs a voltage value V₄₃₅ pertaining to the 435 nm light quantity.

The differential computation processor 27 outputs a differential voltage value Vd that indicates the difference between the 415 nm light quantity and the 435 nm light quantity on the basis of the voltage value V₄₁₅ outputted from the 415 nm light quantity detector 25 and the voltage value V₄₃₅ outputted from the 435 nm light quantity detector 26. In this embodiment, the differential voltage value Vd is found by subtracting the voltage value V₄₃₅ from the voltage value V₄₁₅.

In this embodiment, the 415 nm light quantity detector 25, the 435 nm light quantity detector 26, and the differential computation processor 27 constitute a "voltage value output component" that outputs a voltage value pertaining to the amount of light of a specific wavelength (including a 415 nm wavelength and a 435 nm wavelength).

The controller 28 acquires an NO concentration on the basis of the differential voltage value Vd outputted from the differential computation processor 27. The controller 28 also performs position switching control on the upstream valve 17, the mid-stream valve 18, and the downstream valve 19. The controller 28 also instructs the drive circuit 22 of the timing at which to supply initialization current and measurement current. The configuration and function of the controller 28 will be discussed later.

The memory 29 stores conversion formulas, etc., for defining calibration curves used in acquiring NO concentration on the basis of the differential voltage value Vd outputted from the differential computation processor 27.

### Detecting Element 30

The detecting element 30 comprises a base material and a metal complex of porphyrin or a derivative thereof (hereinafter sometimes referred to simply as "metal complex") that is supported by the base material.

The base material may be a woven cloth, nonwoven cloth, porous material, or the like. The base material may be formed from cellulose; glass fiber; a polymer such as polyester or polypropylene, or the like. An example of a polyester is PET (polyethylene terephthalate). The porphyrin preferably impregnates the base material. For example, the porphyrin may seep into or be kneaded into the base material.

The metal complex is one whose absorption spectrum changes when exposed to a nitrogen oxide.

Derivatives of porphyrin encompass substances in which substituents are bonded to a porphyrin skeleton. Examples of substituents include branchable alkyl groups, alkylene groups, aryl groups, and other such hydrocarbon groups; a methoxy group, ethoxy group, and other such alkoxy groups; halogen groups; a hydroxy group; an amino group; an imino group; a nitro group; and a carbonyl group.

Examples of the metal that forms a coordinate bond with the porphyrin in the metal complex include cobalt, iron, and magnesium.

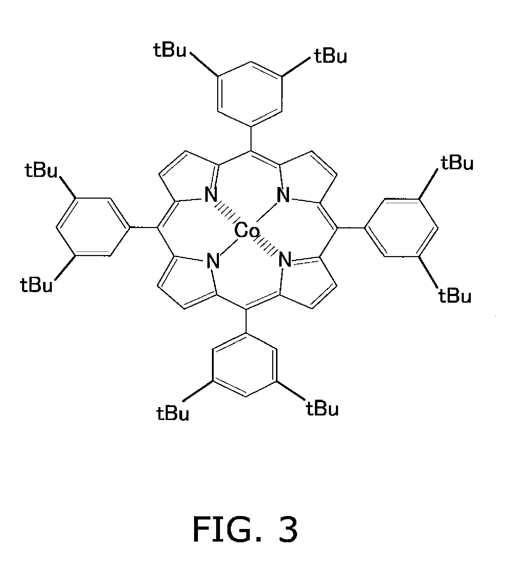

In this embodiment, the detecting element 30 comprises a nonwoven cloth and a metal complex that has permeated the nonwoven cloth by being dripped onto it. The detecting element 30 also comprises as the metal complex the CoT(dtBu)PP shown in FIG. 3, which consists of a cobalt complex of a porphyrin derivative having a tertiary butyl group (t-Bu) located at the 3.5 position of a phenyl group.

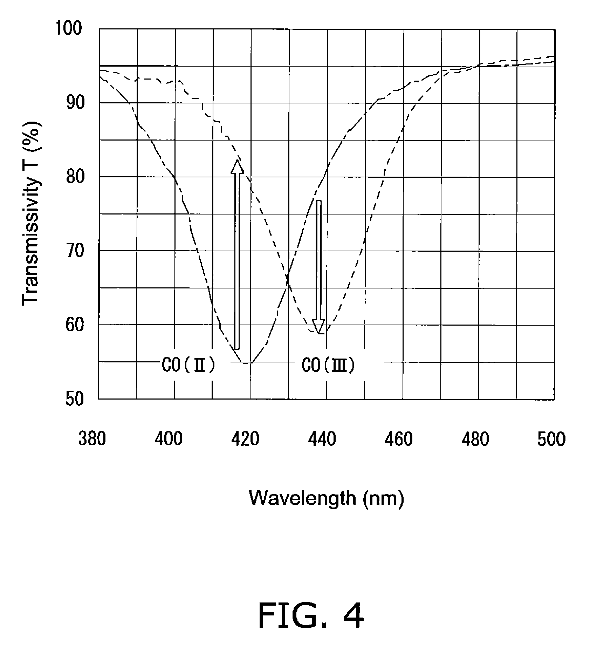

As shown in FIG. 4, the CoT(dtBu)PP has an absorption peak near 415 to 420 nm when the valence of the cobalt is 2 (Co(II)). The valence of cobalt change from divalent to trivalent (Co(III)) when exposed to NO. Co(III) has an absorption peak near 435 to 440 nm. When the detecting element 30 is exposed to the gas being detected, if the NO concentration is high in the gas being detected, there is a large change in the absorption spectrum, and if the NO concentration is low, there is a small change in the absorption spectrum.

Thus, the NO concentration can be measured by comparing the amount of light with a specific wavelength transmitted from the detecting element 30 prior to exposure to the gas being detected, with the amount of light with the specific wavelength transmitted from the detecting element 30 after to exposure to the gas being detected.

In this embodiment, as will be discussed below, the difference between the 415 nm light quantity and the 435 nm light quantity is acquired before and after exposure of the detecting element to the gas being detected, and the NO concentration is acquired on the basis of a conversion formula and the voltage value of this difference.

It is also possible that the wavelength of the light used in detection will vary with the configuration of the porphyrin or derivative thereof (the type of substituents) and the metal element.

Also, in this embodiment, the nitrogen oxide concentration measurement device 10 initializes the detecting element by irradiating the detecting element 30 with light that is stronger than during concentration detection by the LED light source 21. This initialization involves putting the detecting element in a state in which it is not exposed to the substance being detected. In this embodiment, the central metal of the complex is cobalt, and as discussed above, the NO concentration is measured on the basis of the change in the valence of the cobalt from divalent to trivalent. However, even when not exposed to the gas being detected, cobalt will change from Co(II) to Co(III) when the detecting element 30 reacts with O₂ or CO in the air. This change lowers the detection accuracy. Thus, the cobalt is reduced to Co(II) by initialization prior to being exposed to the detection gas.

### Configuration of Controller 28

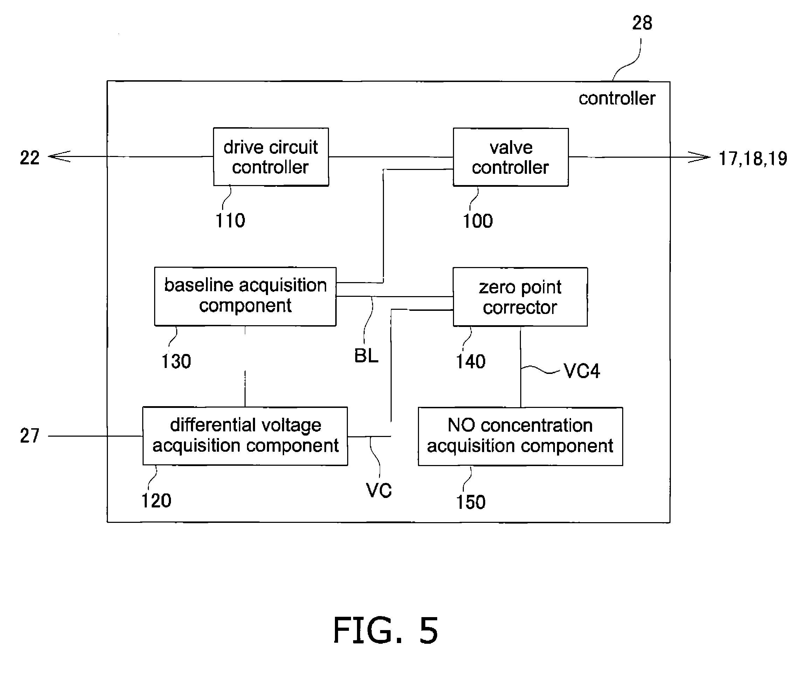

The configuration of the controller 28 pertaining to this embodiment will be described through reference to the drawings. FIG. 5 is a function block diagram of the configuration of the controller 28 pertaining to an embodiment. The configuration of the controller 28 will now be described along with its function.

The controller 28 comprises a valve controller 100, a drive circuit controller 110, a differential voltage acquisition component 120, a baseline acquisition component 130, a zero point corrector 140, and an NO concentration acquisition component 150.

The valve controller 100 executes the above-mentioned position switching control. More specifically, the valve controller 100 uses the upstream valve 17 to block off the path to the chamber 15 while the user is inhaling, and uses the upstream valve 17 to block off the path to the NO scrubber 12 while the user is exhaling. Consequently, after air that has undergone the NO removal treatment by the NO scrubber 12 is inhaled by the user, the exhaled air is sent to the chamber 15.

When the user is exhaling, the valve controller 100 uses the mid-stream valve 18 and the downstream valve 19 to block off the second flow path 16b during a first specific period (such as about 5 seconds; hereinafter referred to as the "first period"). The valve controller 100 uses the mid-stream valve 18 and the downstream valve 19 to block off the first flow path 16a during a specific period that follows the first period (such as about 5 seconds; hereinafter referred to as the "second period"). The valve controller 100 uses the mid-stream valve 18 and the downstream valve 19 to block off the second flow path 16b during a specific period that follows the second period (such as about 5 seconds; hereinafter referred to as the "third period"). Consequently, during the first period exhaled air that has undergone the NO removal treatment by the zero scrubber 13 is sent to the chamber 15, during the second period exhaled air that has not undergone the NO removal treatment is sent to the chamber 15, and during the third period exhaled air that has undergone the NO removal treatment by the zero scrubber 13 is again sent to the chamber 15. Therefore, the detecting element 30 is exposed to exhaled air that has undergone the NO removal treatment during the first period, is exposed to just the exhaled air itself during the second period, and is exposed to exhaled air that has undergone the NO removal treatment during the third period.

The drive circuit controller 110 sends commands to the drive circuit 22 about the timing of supplying the initialization current and measurement current. More specifically, the drive circuit controller 110 instructs the drive circuit 22 to supply initialization current when the detecting element 30 has been installed in the chamber 15. The drive circuit controller 110 instructs the drive circuit 22 to supply measurement current when the user is inhaling. The drive circuit controller 110 instructs the drive circuit 22 to halt the measurement current at the end of the third period.

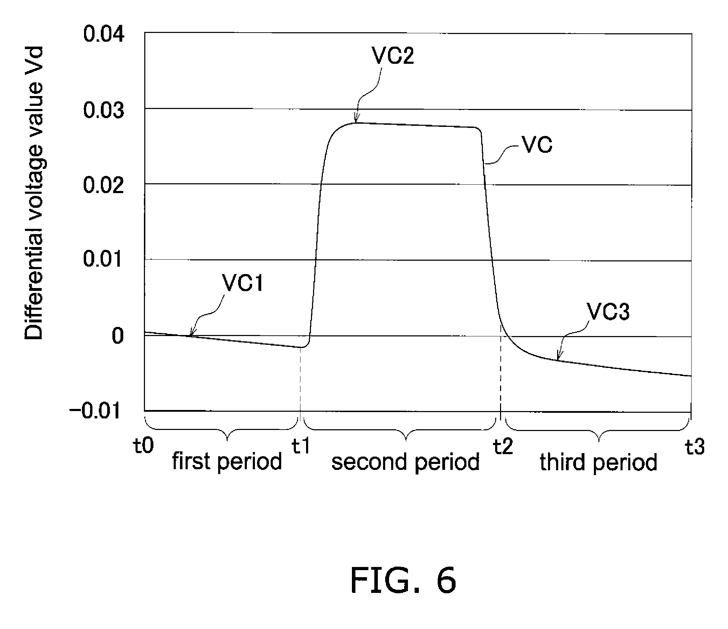

The differential voltage acquisition component 120 acquires in real time the differential voltage value Vd outputted from the differential computation processor 27. FIG. 6 is a graph showing an example of the differential voltage value curve VC, which plots the change in the differential voltage value Vd. As shown in FIG. 6, the differential voltage value curve VC includes a first differential voltage value curve VC1 corresponding to the first period (time t0 to time t1), a second differential voltage value curve VC2 corresponding to the second period (time t1 to time t2), and a third differential voltage value curve VC3 corresponding to the third period (time t2 to time t3). The differential voltage value curve VC is the aggregate of the differential voltage values Vd outputted in real time from the differential voltage acquisition component 120.

In the example shown in FIG. 6, it can be seen that the second differential voltage value curve VC2 gradually decreases as the measurement time passes, and that the second differential voltage value curve VC2 is not stable. Similarly, the first differential voltage value curve VC1 and the third differential voltage value curve VC3 can also be seen to undergo an overall decrease as measurement time passes.

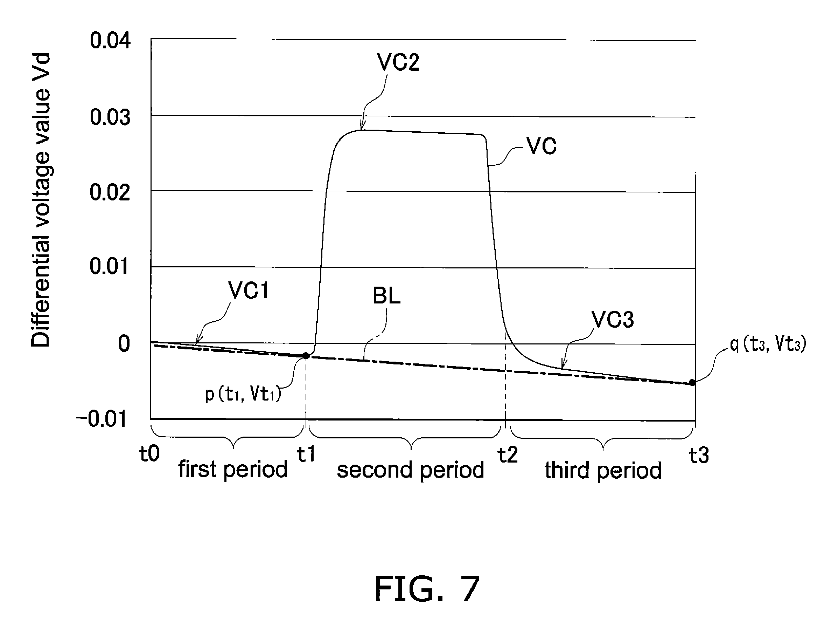

The baseline acquisition component 130 acquires a baseline BL for the zero point correction of the differential voltage value curve VC on the basis of the first differential voltage value curve VC1 and the third differential voltage value curve VC3. FIG. 7 is a graph showing an example of the baseline BL. In this embodiment, the baseline BL is interpolated on the basis of a first differential voltage value Vₜ₁ at the end time t1 of the first period, and a third differential voltage value Vₜ₃ at the end time t3 of the third period. The baseline BL is a straight line connecting a coordinate p (first differential voltage value Vₜ₁, time t1) and a coordinate q (third differential voltage value Vₜ₃, time t3).

The zero point corrector 140 subjects the differential voltage value curve VC to zero point correction on the basis of the baseline BL. As a result, the zero points are matched for a plurality of differential voltage values included in the second differential voltage value curve VC2 (hereinafter referred to as "a plurality of second differential voltage values Vₜ₂). This zero point correction of the plurality of second differential voltage values Vₜ₂ produces a fourth differential voltage value curve VC4 that includes a plurality of fourth differential voltage values.

The NO concentration acquisition component 150 acquires the NO concentration of exhaled air according to the fourth differential voltage value curve VC4 produced by the zero point corrector 140. More specifically, the NO concentration acquisition component 150 uses a conversion formula expressing a calibration curve to convert the fourth differential voltage value curve VC4, thereby acquiring the transition in the NO concentration. The NO concentration acquisition component 150 acquires the average value for the period in which the NO concentration is stable as the concentration in which NO is contained in the exhaled air.

### Action and Effect

(1) The nitrogen oxide concentration measurement device 10 pertaining to an embodiment comprises the zero scrubber 13 disposed within the gas flow path 16. The zero scrubber 13 subjects exhaled air flowing through the gas flow path 16 from the mouthpiece 11 toward the chamber 15 to a NO removal treatment during a first period, out of a first period and a second period. The controller 28 acquires the concentration in which the NO is contained in the exhaled air on the basis of the differential voltage value Vd outputted in the second period, which uses as a reference the differential voltage value Vd outputted in the first period.

Thus, the controller 28 performs zero point correction using exhaled air that has undergone the NO removal treatment by the zero scrubber 13. Therefore, there is less fluctuation in the gas component after the NO removal treatment than when a zero point correction-use gas is produced by subjecting outside air to the NO removal treatment, so the concentration in which NO is contained in the exhaled air can be measured more accurately.

Also, since there is no need to provide a compressor or the like for taking in outside air, the configuration of the nitrogen oxide concentration measurement device 10 can be simplified.

(2) The mid-stream valve 18 (an example of a "first flow path converter") disposed at the branch point between the first flow path 16a and the second flow path 16b within the gas flow path 16 closes the second flow path during the first period, and closes the first flow path during the second period.

Therefore, while the user is inhaling, exhaled air that has undergone the NO removal treatment and exhaled air that has not undergone this treatment can alternately flow to the chamber 15. Accordingly, there is no need to provide a storage room for temporarily holding exhaled air apart from the chamber 15. This affords further simplification of the configuration of the nitrogen oxide concentration measurement device 10.

(3) The nitrogen oxide concentration measurement device 10 comprises a downstream valve 19 (an example of a "second flow path converter") disposed at the merge point between the first flow path 16a and the second flow path 16b within the gas flow path 16. The downstream valve 19 closes the second flow path during the first period, and closes the first flow path during the second period.

Therefore, less of the exhaled air going from the second flow path 16b toward the chamber 15 comes into contact with the zero scrubber 13 within the first flow path 16a. Accordingly, the concentration in which NO is contained in the exhaled air fluctuates less within the gas flow path 16, so the NO concentration in the exhaled air can be measured more accurately.

(4) With the nitrogen oxide concentration measurement device 10 pertaining to an embodiment, the controller 28 subjects the second differential voltage value curve VC2 acquired in the second period to zero point correction on the basis of the first differential voltage value curve VC1 acquired in the first period and the third differential voltage value curve VC3 acquired in the third period. The controller 28 acquires the NO concentration in the exhaled air according to a fourth voltage value produced by zero point correction.

The inventors conducted diligent study into the cause of fluctuation ("decrease" in this embodiment) in the second differential voltage value curve VC2 as measurement time passes. As a result, it was discovered that the first differential voltage value curve VC1 and the third differential voltage value curve VC3 fluctuate along with measurement time (see FIG. 6), and that this fluctuation tends to be different each time measurement is performed. This is because the irradiation light during measurement creates tiny changes in the dissociation equilibrium between the oxygen in the exhaled air and the cobalt in the detecting element 30. Accordingly, there is the risk that variance will turn up in the NO concentration measurement results if the second differential voltage value curve VC2 undergoes zero point correction on the basis of the first differential voltage value curve VC1 alone.

In view of this, in this embodiment the second differential voltage value curve VC2 is subjected to zero point correction on the basis of the baseline BL acquired according to the first differential voltage value curve VC1 and the third differential voltage value curve VC3, as discussed above. Specifically, since the differential voltage value curve VC is accurately corrected for zero point by interpolating the baseline BL corresponding to the change in the zero point, the NO concentration can be measured more accurately.

(5) The controller 28 pertaining to this embodiment acquires the first differential voltage value Vₜ₁ at the end time t1 of the first period, and the third differential voltage value Vₜ₃ at the end time t3 of the third period. The controller 28 acquires the baseline BL on the basis of the first differential voltage value Vₜ₁ and the third differential voltage value Vₜ₃.

The first differential voltage value Vₜ₁ here is the voltage value at the end time t1 closest to the second period, and the third differential voltage value Vₜ₃ is the voltage value at the point when the differential voltage value Vd is most stable after the second period. Therefore, the baseline BL can accurately correspond to changes in the zero point, so the NO concentration can be measured more accurately.

(6) The differential computation processor 27 outputs a voltage value indicating the difference between the 415 nm light quantity and the 435 nm light quantity. Therefore, smaller changes in the quantity of light can be detected than when just the 415 nm light quantity or the 435 nm light quantity is detected, so the NO concentration can be measured more accurately.

### Other Embodiments

An embodiment of the present invention was described above, but the present invention is not limited to or by the above embodiment, and various modifications are possible without departing from the gist of the invention.
(A) In the above embodiment, the controller 28 acquired the differential voltage values Vd in real time from the differential computation processor 27, but this is not the only option. The controller 28 may acquire just a portion of the differential voltage values Vd outputted from the differential computation processor 27. For instance, the controller 28 may perform zero point correction on one differential voltage value Vd acquired at a specific time in the second period on the basis of two differential voltage values Vd acquired at a specific time in the first period and a specific time in the third period. Specifically, the controller 28 may sometimes not acquire the differential voltage value curve VC.
(B) In the above embodiment, the controller 28 acquired the baseline BL on the basis of the first differential voltage value Vₜ₁ at the end time t1 of the first period and the third differential voltage value Vₜ₃ at the end time t3 of the third period, but this is not the only option. For instance, the controller 28 may acquire the average of all the plurality of differential voltage values in the first period, instead of the first differential voltage value Vₜ₁. Similarly, the controller 28 may acquire the average of all the plurality of differential voltage values in the third period, instead of the third differential voltage value Vₜ₃.

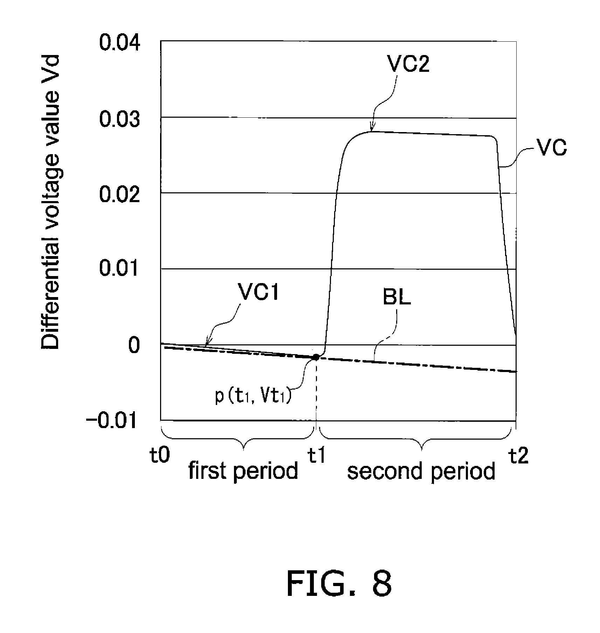

Also, the controller 28 can acquire the baseline BL on the basis of a plurality of differential voltage values in the first period and third period, rather than on the basis of a representative differential voltage value in the first period and in the third period. For example, the controller 28 can acquire as the baseline BL an approximation line or approximation curve obtained from a plurality of differential voltage values among the first and third periods by the method of least squares.
(C) In the above embodiment, the base material of the detecting element was said to transmit light, but this is not the only option. The base material of the detecting element may be configured such that it reflects light, and the nitrogen oxide concentration measurement device may be configured such that a variety of signals is obtained by detecting the amount of reflected light, rather than the amount of light transmitted from the detecting element. In this case, the base material of the sensor can be one that reflects light, such as alumina, silicon, silicon carbide, gallium arsenide, a ceramic, a plastic, or a metal. Therefore, the 415 nm light quantity detector 25 and the 435 nm light quantity detector 26 should be able to detect light emitted from the detecting element.
(D) In the above embodiment, the NO concentration was measured on the basis of both 415 nm light and 435 nm light, but this is not the only option. The nitrogen oxide concentration measurement device 10 may measure NO concentration on the basis of just the change in the amount of light of a single wavelength.
(E) In the above embodiment, initialization was executed by irradiating the detecting element 30 with light, but the initialization may instead be executed by applying heat to the detecting element 30.
(F) In the above embodiment, the baseline BL was set linearly, but may instead be a curve.
(G) Although not touched upon directly in the above embodiment, the nitrogen oxide concentration measurement device 10 may comprise a flow meter for measuring the flow of exhaled air, a thermometer for measuring the temperature, a display monitor for displaying the measured NO concentration, or the like.
(H) In the above embodiment, the baseline acquisition component 130 acquired the baseline BL for the zero point correction of the differential voltage value curve VC on the basis of the first differential voltage value curve VC1 and the third differential voltage value curve VC3, but this is not the only option. The baseline acquisition component 130 may acquire the baseline BL on the basis of just the first differential voltage value curve VC1. In this case, as shown in FIG. 8, at the end time t2 of the second period, the opening and closing control by the valve controller 100 and the supply of gas to the chamber 15 are ended, and the drive circuit controller 110 instructs the drive circuit 22 to halt the flow of measurement current at the end time t2 of the second period. Here again, since zero point correction can be performed using exhaled air that has undergone the NO removal treatment, the concentration of NO contained in the exhaled air can be measured more accurately.

Again in this case, the method for setting the baseline BL can be selected as needed, but the baseline BL may be an approximation line or approximation curve obtained from a plurality of differential voltage values Vd in the first period by the method of least squares, or the baseline BL may be the average of a plurality of differential voltage values Vd in the first period.

### INDUSTRIAL APPLICABILITY

The nitrogen oxide concentration measurement device of the present invention can accurately measure the concentration of NO contained in exhaled air, and therefore can be widely applied to sensors that measure the concentration of nitrogen oxides.

### REFERENCE SIGNS LIST

- 10: nitrogen oxide concentration measurement device
- 11: mouthpiece
- 12: NO scrubber
- 13: zero scrubber (an example of a "NO removal unit")
- 14: dryer
- 15: chamber
- 16: gas flow path
- 16a: first flow path
- 16b: second flow path
- 17: upstream valve
- 18: mid-stream valve (an example of a "first flow path switching component")
- 19: downstream valve (an example of a "second flow path switching component")
- 21: LED light source (an example of a "light source")
- 22: drive circuit
- 23: lens
- 24: dichroic mirror
- 25: 415 nm light quantity detector
- 26: 435 nm light quantity detector
- 27: differential computation processor
- 28: controller
- 29: memory
- 30: detecting element
- 100: valve controller
- 110: drive circuit controller
- 120: differential voltage acquisition component
- 130: baseline acquisition component
- 140: zero point corrector
- 150: NO concentration acquisition component

## Claims

1. A nitrogen oxide concentration measurement device, comprising:
a detecting element containing a metal complex of porphyrin or a derivative thereof;
a mouthpiece for taking in exhaled air;
a chamber for housing the detecting element;
a gas flow path that communicates with the mouthpiece and the chamber;
a NO removal unit that is disposed within the gas flow path, and that subjects exhaled air flowing through the gas flow path from the mouthpiece toward the chamber to a NO removal treatment in a first period, out of the first period and a second period that follows the first period;
a light source that shines light on the detecting element during the first and second periods;
a voltage value output component that outputs, during the first and second periods, a voltage value indicating the amount of light having a specific wavelength out of all the light emitted from the detecting element; and
a controller that acquires the concentration of NO contained in the exhaled air on the basis of the voltage value outputted from the voltage value output component in the second period, which uses as a reference the voltage value outputted from the voltage value output component in the first period.

2. The nitrogen oxide concentration measurement device according to Claim 2,
wherein the gas flow path has a first flow path that houses the NO removal unit, a second flow path that is linked to both ends of the first flow path, and a first flow path switching component that is disposed at the branch point of the first flow path and the second flow path, and
the first flow path switching component closes the second flow path in the first period, and closes the first flow path in the second period.

3. The nitrogen oxide concentration measurement device according to Claim 2,
wherein the gas flow path has a second flow path switching component that is disposed at the merge point of the first flow path and the second flow path, and
the second flow path switching component closes the second flow path in the first period, and closes the first flow path in the second period.

4. The nitrogen oxide concentration measurement device according to Claim 1,
wherein the NO removal unit subjects exhaled air to the NO removal treatment in a third period that follows the second period,
the voltage value output component outputs a voltage value indicating the amount of light having a specific wavelength out of all the light emitted from the detecting element, in the third period following the first and second periods, and
the controller acquires first, second, and third voltage values outputted from the voltage value output component in the first, second, and third periods, and acquires the concentration of NO contained in the exhaled air according to a fourth voltage value produced by correcting the second voltage value on the basis of the first and third voltage values.

5. The nitrogen oxide concentration measurement device according to Claim 4,
wherein the controller acquires as the first voltage value the voltage value outputted from the voltage value output component at the end of the first period, and acquires as the third voltage value the voltage value outputted from the voltage value output component at the end of the third period.

6. The nitrogen oxide concentration measurement device according to Claim 4,
wherein the controller acquires as the first voltage value the average of a plurality of voltage values outputted from the voltage value output component during the first period, and acquires as the third voltage value the average of a plurality of voltage values outputted from the voltage value output component during the third period.

7. The nitrogen oxide concentration measurement device according to Claim 4,
wherein the specific wavelength includes a first wavelength and a second wavelength that is different from the first wavelength, and
the voltage value output component outputs a voltage value indicating the difference between the amount of light having the first wavelength and the light of light having the second wavelength.
